# EUROPEAN PATENT APPLICATION

(11) **EP 4 599 928 A1**
(43) Date of publication of application: **13.08.2025**
(21) Application number: 24875759.3
(22) Date of filing: 05.09.2024
(51) Int. Cl.: B01J 8/00, B01J 19/00, C07C 2/08, C07C 11/107

(54) **APPARATUS FOR PRODUCING OLIGOMERS**

(30) Priority: 28.11.2023 KR 20230167898
(71) Applicant: LG Chem, Ltd., Seoul 07336 (KR)
(72) Inventor: SONG, Jong Hun, Daejeon 34122 (KR); LEE, Jeong Seok, Daejeon 34122 (KR); BAEK, Se Won, Daejeon 34122 (KR); HWANG, Moon Sub, Daejeon 34122 (KR); PAK, Geun Tae, Daejeon 34122 (KR)
(74) Representative: Hoffmann Eitle
(86) International application number: PCT/KR2024/013402
(87) International publication number: WO 2025/116225

(57) **Abstract**

The present disclosure provides an apparatus for preparing an oligomer, including: a reactor that includes an upper gas region and a lower liquid region and performs an oligomerization reaction by receiving an ethylene gas and a solvent to the liquid region; an overflow pipe that is provided on an outer side wall of the reactor and connected to a height corresponding to a boundary between the gas region and the liquid region of the reactor to discharge a liquid stream containing an oligomer product; a gas-liquid separator that is connected to the overflow pipe and separates gas from the discharged liquid stream; a liquid level gauge that is provided in the gas-liquid separator; and a liquid discharge pipe that is connected to a lower portion of the gas-liquid separator and discharges the gas-separated liquid stream.

## Description

### [Technical Field]

### CROSS-REFERENCE TO RELATED APPLICATION(S)

This application claims priority to and the benefit of Korean Patent Application No. 10-2023-0167898 filed on November 28, 2023, which is hereby incorporated by reference in their entirety.

### TECHNICAL FIELD

The present disclosure relates to an apparatus for preparing an oligomer, and more particularly, to an apparatus for preparing an oligomer capable of preventing pipe clogging by stably maintaining a liquid level of a reactor during the preparation of the oligomer and minimizing the companying of gas hole up in a liquid discharged from the reactor.

### [Background Art]

Alpha-olefins are an important material used in comonomers, detergents, lubricants, plasticizers, and the like, and are commercially widely used. Among them, 1-hexene and 1-octene have been widely used as comonomers for controlling a density of polyethylene at the time of preparing linear low-density polyethylene (LLDPE).

These alpha olefins may be prepared by performing an oligomerization reaction in a state where ethylene is dissolved in a solvent in the presence of a catalyst, and the oligomerization reaction may be performed, for example, in a bubble column reactor.

The bubble column reactor causes a raw material gas to flow into a liquid region containing a solvent, a catalyst, etc., through a distribution device installed at a lower portion thereof and to be dispersed while rising in the form of bubble, and performs an oligomerization reaction by mixing the dispersed gas within the liquid region while generating turbulence.

In this case, since the liquid level in the reactor affects the control of reaction conditions such as pressure and temperature, a catalyst residence time, the resulting ethylene conversion amount, a heating value, etc., the liquid level in the reactor should be maintained constant. To this end, it is very important to indicate a liquid height in the reactor.

As the solvent, the ethylene dissolved in the solvent, and the liquid substance (oligomer) generated by conversion from the ethylene exist together in the liquid region where the oligomerization reaction takes place in the reactor, the composition of the liquid changes, so a density of the liquid after the reaction changes compared to the liquid before the reaction. In particular, the density of the entire liquid volume may be greatly reduced by the amount of accompanying of gas hole up in the liquid. In this way, the density is affected by the characteristics (temperature, pressure, composition, viscosity, etc.) of the liquid substance in the reactor.

In order to measure the liquid level in the reactor during the preparation of the oligomer, a differential pressure type level transmitter (LT) is usually applied, but the liquid density may change depending on the degree of reaction, and thus, the liquid level may be incorrectly indicated. When the liquid level incorrectly indicated by the differential pressure type LT occurs significantly, it is difficult to maintain the actual liquid level in the reactor constant.

In addition, when a large amount of gas hole up is accompanied in the liquid substance produced by the oligomerization reaction and are transferred to a liquid discharge pipe connected to a lower portion of the reactor, a serious clogging phenomenon may occur due to an increase in two-phase in the pipe.

### [Disclosure]

### [Technical Problem]

The present disclosure is intended to solve the problems mentioned in the background art, and provides an apparatus for preparing an oligomer capable of preventing pipe clogging by stably maintaining a liquid level of a reactor during the preparation of the oligomer and minimizing the accompanying of gas hole up in a liquid discharged from the reactor.

### [Technical Solution]

In one general aspect, an apparatus for preparing an oligomer includes: a reactor that includes an upper gas region and a lower liquid region and performs an oligomerization reaction by receiving an ethylene gas and a solvent to the liquid region; an overflow pipe that is provided on an outer side wall of the reactor and connected to a height corresponding to a boundary between the gas region and the liquid region of the reactor to discharge a liquid stream containing an oligomer product; a gas-liquid separator that is connected to the overflow pipe and separates a gas from the liquid stream; a liquid level gauge that is provided in the gas-liquid separator; and a liquid discharge pipe that is connected to a lower portion of the gas-liquid separator and discharges the gas-separated liquid stream.

### [Advantageous Effects]

According to the apparatus for preparing an oligomer according to the present disclosure, it is possible to suppress the accompanying of gas hole up by transferring a liquid stream that has risen above a boundary height from a reactor to a gas-liquid separator while maintaining the pressure of the reactor and the gas-liquid separator to be the same through an overflow pipe provided at a boundary between a gas region and a liquid region in the reactor, thereby minimizing a change in density of the liquid. Thereafter, by measuring a height of the liquid in which the change in density is minimized in the gas-liquid separator to control the amount of liquid discharged, it is possible to stably maintain the liquid level in the reactor.

In addition, by suppressing the amount of gas hole up in the gas-separated liquid stream discharged through the gas-liquid separator, it is possible to smooth a flow of oligomer products in a liquid discharge pipe, prevent a clogging phenomenon, and reduce the discharge of ethylene gas to a downstream stream.

### [BRIEF DESCRIPTION OF THE DRAWINGS]

FIG. 1 is a diagram schematically illustrating a process flow using an apparatus for preparing an oligomer according to an embodiment of the present disclosure.
FIGS. 2 and 3 are diagrams schematically illustrating a process flow using an apparatus for preparing an oligomer according to a comparative example.

### [Best Mode]

Terms and words used in the present specification and claims are not to be construed as a general or dictionary meaning, and are to be construed as meaning and concepts meeting the technical spirit of the present disclosure based on a principle that the inventors may appropriately define the concepts of terms in order to describe their own inventions in the best mode.

A term "including" or "containing" used herein concretely indicates specific properties, regions, integer numbers, steps, operations, elements, or components, and is not to exclude presence or addition of other specific properties, regions, integer numbers, steps, operations, elements, components, or a group thereof.

The term 'stream' used herein may mean a flow of a fluid in a process, and may also mean a fluid itself flowing in a pipe. Specifically, the stream may mean both the fluid itself and the flow of the fluid flowing within the pipe connecting each device. In addition, the fluid may include any at least one component of a gas, a liquid, or a solid.

Unless otherwise specified, the term 'upper portion' as used herein means a point at a height of 0 to 50% downward from a top of a device, and may specifically mean the top (top of column). In addition, the term 'lower portion' means a point at a height of 50 to 100% downward from the top of the device, and may specifically mean the bottom (bottom of column).

Unless otherwise specified, the term 'side stream' as used herein may mean a stream discharged at a height of 25 to 80% downward from the top of the device or a height of 40 to 70%.

In addition, the "pressure" mentioned herein means a gauge pressure measured based on atmospheric pressure.

Hereinafter, the present disclosure will be described in detail with reference to the accompanying drawings.

An embodiment of the present disclosure relates to an apparatus for preparing an oligomer.

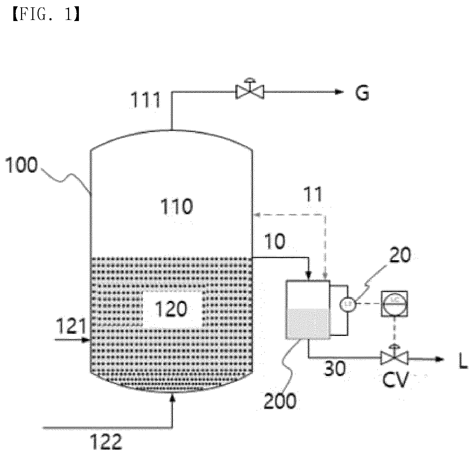

Referring to FIG. 1, the apparatus according to the present disclosure includes a reactor 100 that performs an oligomerization reaction; an overflow pipe 10 that is connected to the reactor to discharge a liquid stream containing an oligomer product; a gas-liquid separator 200 that is connected to the overflow pipe; a liquid level gauge 20 that is provided in the gas-liquid separator 200; and a liquid discharge pipe 30 that is connected to a lower portion of the gas-liquid separator 200.

The reactor 100 includes an upper gas region 110 and a lower liquid region 120, and in the liquid region 120, an oligomerization reaction of a monomer may be performed in a liquid state in which an ethylene gas is dissolved in a solvent. The solvent may be supplied through a solvent inlet pipe 121 together with a catalyst, and the ethylene gas may be supplied from a gas inlet pipe 122.

As the reactor, a continuous stirred-tank reactor, a plug flow reactor, a bubble column reactor, etc., may be used.

For example, a raw material gas flows into a liquid region containing a solvent, a catalyst, etc., through a distribution device installed at a lower portion of a bubble column reactor and is dispersed while rising in the form of bubble, and the dispersed gas may perform an oligomerization reaction by being mixed within the liquid region while generating turbulence.

The oligomerization reaction may refer to a reaction in which the monomers are oligomerized. Depending on the number of monomers that are polymerized, the oligomerization reaction is called trimerization or tetramerization, and is collectively called multimerization. The alpha olefins such as 1-hexene and 1-octene may be prepared, for example, through trimerization or tetramerization of ethylene.

The ethylene gas supplied to the reactor 100 may be a stream containing ethylene (C2) separated from naphtha thermal cracking.

The solvent for dissolving the ethylene gas may include one or more selected from the group consisting of n-pentane, n-hexane, n-heptane, cyclohexane, methyl cyclohexane, octane, cyclooctane, decane, dodecane, benzene, xylene, 1,3,5-trimethylbenzene, toluene, ethylbenzene, chlorobenzene, dichlorobenzene, and trichlorobenzene. In some cases, a mixture of two or more of those described above may be used as the solvent. Therefore, the ethylene gas may be liquefied at a higher temperature, and a dissolution rate at which the ethylene gas is dissolved in the solvent may be improved.

In the oligomerization reaction of the ethylene, a compound containing a transition metal may be used as a catalyst to promote reaction activity. For example, the catalyst may be a compound containing one or more selected from the group consisting of, for example, chromium(III) acetylacetonate, chromium(III) chloride tetrahydrofuran, chromium(III) 2-ethylhexanoate, chromium(III) tris(2,2,6,6-tetramethyl-3,5-heptanedionate), chromium (III) benzoylacetonate, chromium (III) hexafluoro-2,4-pentaindionate, chromium (III) acetate hydroxide, chromium (III) acetate, chromium(III) butyrate, chromium(III) pentanoate, chromium(III) laurate, and chromium(III) stearate.

In addition, a cocatalyst may be additionally used to increase the activity of the above catalyst. The cocatalyst may include one or more selected from the group consisting of, for example, trimethyl aluminum, triethyl aluminum, triisopropyl aluminum, triisobutyl aluminum, ethylaluminum sesquichloride, diethylaluminum chloride, ethyl aluminum dichloride, methylaluminoxane, modified methylaluminoxane, and borate.

The oligomerization reaction may be operated under conditions commonly applied in the relevant field, and may be performed at a temperature of 30°C to 150°C or 50°C to 120°C and a pressure of 20 bar to 65 bar or 20 bar to 40 bar, for example.

When this oligomerization reaction is performed, a liquid stream containing an oligomer product polymerized with ethylene, a by-product, a solvent, an unreacted gas dissolved in the solvent, etc., may exist in the lower liquid region 120 of the reactor 100, and the unreacted ethylene gas that is not dissolved in the solvent and thus does not participate in the oligomerization reaction may rise to the upper gas region 110. The gas region of the reactor includes an upper pipe 111 that is configured to discharge a gas stream containing an unreacted ethylene gas.

The liquid stream existing in the liquid region 120 of the reactor may have a density changing depending on the amount of liquid oligomer product generated by conversion from the ethylene and the amount of gas hole up derived from the unreacted gas dissolved in the solvent, and when an incorrect instruction occurs during the measurement of the liquid level due to the change in density, it may be difficult to stably maintain the liquid level in the reactor.

To overcome the problem, in the present disclosure, the overflow pipe 10 is provided at a height corresponding to a boundary between the gas region 110 and the liquid region 120 on an outer side wall of the reactor, and is connected to the gas-liquid separator 200, so a liquid stream containing an oligomer product obtained in the reactor 100 is transferred to the gas-liquid separator 200 through the overflow pipe 10, and then the liquid level of the gas-separated liquid stream in the gas-liquid separator 200 is measured.

More specifically, the overflow pipe 10 enables the movement of the liquid stream by the overflow at the boundary between the gas region and the liquid region of the reactor 100, and at the same time, pressure equilibrium between the reactor 100 and the gas-liquid separator 200 may be achieved. That is, the pressures of the reactor 100 and the gas-liquid separator 200 are maintained the same by the overflow pipe 10, so that the gas is in equilibrium, and only the liquid that has risen above the boundary height may flow out from the reactor 100 to the gas-liquid separator 200.

When the overflow pipe is not provided, it is difficult to maintain the pressures of the reactor and the gas-liquid separator the same. For example, when connecting the pipe to the lower liquid region of the reactor to transfer the liquid stream to the gas-liquid separator, a pressure gauge and a pressure control valve will be additionally required to control the pressure of the gas-liquid separator. In addition, when an upper gas stream of the gas-liquid separator is injected into an upper gas region of the reactor, the mixing of the two streams may be restricted by each pressure relationship, and since the upper gas stream of the gas-liquid separator is a saturated gas, there is a possibility that condensation may occur.

The overflow pipe 10 may have a sufficient size to effectively achieve the movement by overflow of the liquid stream and the pressure equilibrium of the gas stream, and there is no particular limitation on an inner diameter and length thereof as long as a flow rate is not restricted when the liquid is discharged through the overflow pipe 10.

The liquid stream transferred from the reactor 100 to the gas-liquid separator 200 through the overflow pipe 10 may suppress the accompanying of the gas hole up, thereby minimizing the change in density of the liquid due to the gas hole up.

In addition, the apparatus for preparing an oligomer according to the present disclosure may further include a pressure maintenance pipe 11 connecting the gas region of the reactor 100 and the gas region of the gas-liquid separator 200 to each other.

The pressure maintenance pipe 11 may be used to more effectively uniform the pressures of the reactor 100 and the gas-liquid separator 200, and may be designed in an ordinary manner in the relevant field.

The gas-liquid separator 200 separates the gas contained in the liquid stream transferred through the overflow pipe 10, the separated gas may be mixed with the gas existing in the reactor 100 through the overflow pipe 10 or the pressure maintenance pipe 11, and the gas-separated liquid stream remains at the lower portion of the gas-liquid separator 200.

The height of the gas-separated liquid stream existing in the gas-liquid separator 200 is measured by the liquid level gauge 20 provided in the gas-liquid separator 200.

The above-mentioned liquid level gauge may be a differential pressure type level transmitter (LT) commonly used in the relevant field, but is not limited thereto.

The accompanying of the gas hole up is suppressed in the liquid transferred to the gas-liquid separator 200, and the composition of the liquid within the gas-liquid separator 200 does not change, so the change in density is not large. Therefore, unlike the reactor 100 where the liquid composition changes, the possibility of occurrence of the incorrect instruction is low when measuring the liquid level of the gas-separated liquid region in the gas-liquid separator 200. For example, the liquid level measurement value may have an error of less than 5% with the actual liquid level in the gas-liquid separator.

Therefore, by measuring the height of the liquid with the suppressed change in density using the liquid level gauge 20 provided in the gas-liquid separator 200, the liquid level in the reactor 100 may be stably maintained by discharging the liquid stream in the gas-liquid separator 200 according to the signal of the liquid level gauge 20.

The gas-separated liquid stream in the gas-liquid separator 200 may be discharged through the liquid discharge pipe 30 connected to the lower portion of the gas-liquid separator 200. In this case, the amount of gas-separated liquid stream discharged may be controlled by controlling the opening and closing of the control valve CV provided in the liquid discharge pipe 30 according to the signal of the liquid level gauge 20.

In addition, by minimizing the amount of gas hole up in the gas-separated liquid stream discharged through the gas-liquid separator, it is possible to smooth a flow of oligomer products in a liquid discharge pipe, prevent a clogging phenomenon, and reduce the discharge of ethylene gas to a downstream stream.

Thereafter, the gas-separated liquid stream discharged through the liquid discharge pipe 30 connected to the lower portion of the gas-liquid separator 200 may be supplied to a separation tower (not illustrated), and the unreacted gas, solvent, etc., may be additionally separated in the separation tower to obtain the purified oligomer product.

If necessary, the apparatus for preparing an oligomer may further include devices necessary for preparing an oligomer, such as a condenser, a reboiler, a pump, a cooler, a filter, a stirrer, a compressor, and a mixer.

### [Mode for Invention]

Hereinafter, the present disclosure will be described in more detail through Examples. However, the following Examples are provided in order to exemplify the present disclosure, it is apparent to those skilled in the art that various modifications and alterations may be made without departing from the scope and spirit of the present disclosure, and the scope of the present disclosure is not limited to these Examples.

### Comparative Example 1:

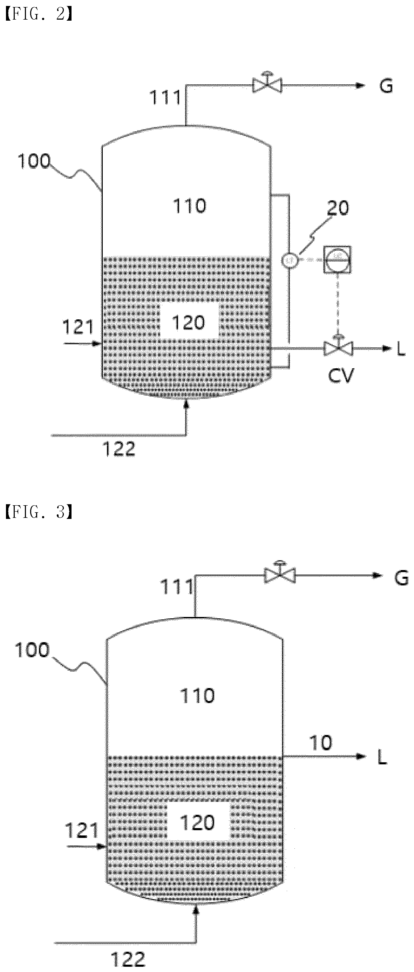

The pilot system as illustrated in FIG. 2 was configured to measure the liquid level in the reactor 100 during the preparation of the oligomer.

First, the solvent, the catalyst, and the ethylene gas were supplied from the lower liquid region 120 of the reactor 100 to perform the oligomerization reaction.

The height of the liquid stream containing the oligomer product obtained by the reaction was measured using a differential pressure type level transmitter (LT) provided in the reactor 100, and the liquid stream was discharged from the lower portion of the reactor 100. The amount of liquid stream discharged was controlled using the control valve CV according to the liquid level measurement value.

### Comparative Example 2:

The pilot system as illustrated in FIG. 3 was configured to measure the liquid level in the reactor 100 during the preparation of the oligomer.

First, the solvent, the catalyst, and the ethylene gas were supplied from the lower liquid region 120 of the reactor 100 to perform the oligomerization reaction.

The liquid stream that contains the oligomer product obtained by the reaction and has risen above the boundary height was discharged through the overflow pipe 10 connected to the height corresponding to the boundary between the upper gas region 110 and the lower liquid region 120 of the reactor 100, thereby maintaining the liquid level in the reactor 100.

Example 1:

The pilot system as illustrated in FIG. 1 was configured to manufacture the oligomer, and the liquid level was measured in the gas-liquid separator 200 installed outside the reactor 100.

First, the solvent, the catalyst, and the ethylene gas were supplied from the lower liquid region 120 of the reactor 100 to perform the oligomerization reaction.

The liquid stream that contains the oligomer product obtained by the reaction and has risen above the boundary height was discharged to the gas-liquid separator 200 through the overflow pipe 10 connected to the height corresponding to the boundary between the upper gas region 110 and the lower liquid region 120 of the reactor 100. In this case, the pressure of the reactor 100 and the gas-liquid separator 200 was maintained the same.

The liquid level in the gas-liquid separator 200 was measured using the differential pressure LT provided in the gas-liquid separator 200, and the gas-separated liquid stream was discharged through the liquid discharge pipe 30 connected to the lower portion of the gas-liquid separator 200. According to the liquid level measurement value, the amount of the gas-separated liquid stream discharged was controlled using the control valve CV provided in the liquid discharge pipe 30.

The results of the liquid level measurement and the liquid maintenance of the reactor according to the above examples and comparative examples were shown in Table 1 below.

**[Table 1]**

| | Liquid leakage inside reactor | Liquid level measuremen t target | Liquid maintenance result |
|---|---|---|---|
| Compar ative Exampl e 1 | Lower pipe | Reactor | Increase in liquid level of reactor |
| Compar ative Exampl e 2 | Overflow pipe | - | Maintain liquid level of reactor /discharge large amount of gas through overflow pipe |
| Exampl e 1 | Overflow pipe | Gas-liquid separator | Maintain liquid level of reactor/maintain liquid level of gas-liquid separator |

As can be seen in Table 1 above, in Example 1, the liquid level was measured by transferring only the liquid, which has risen above the connection height of the overflow pipe, to the gas-liquid separator while maintaining the pressures of the reactor and the gas-liquid separator to be the same through the overflow pipe provided at the boundary between the gas region and the liquid region of the reactor, and as a result, the liquid level in the reactor was stably maintained and there was almost no change in density due to the gas hole up in the liquid in the gas-liquid separator, so the incorrect instruction of the differential pressure LT was minimized. Accordingly, the liquid level of the gas-liquid separator was maintained constant through the liquid level control valve CV connected to the differential pressure LT.

In addition, by minimizing the amount of gas hole up in the gas-separated liquid stream discharged through the gas-liquid separator, it is possible to smooth the flow of oligomer products in the liquid discharge pipe, prevent the clogging phenomenon, and reduce the discharge of ethylene gas to the downstream stream.

On the other hand, Comparative Example 1 directly measured the liquid of the reactor 100, so the incorrect instruction occurred that decreased the LT indication at the same height. Since the gas hole up accompanying in the liquid inside the reactor, i.e., the gas hold-up space distributed as the oligomer is generated, increases, the liquid level is measured in a state where the density of the liquid decreases. As a result, even if the liquid level exists at the same height, the differential pressure decreases, so the LT indication value is displayed as the decrease in liquid level. The control valve was closed to control the liquid level by the incorrect instruction, so the actual liquid level in the reactor rose. Due to this increase in the liquid level in the reactor, the catalyst residence time in the reactor become longer, there were limits to the control of the amount of products and the heating value, the clogging phenomenon due to polymer occurred in the pipe through which the gas-separated liquid stream is discharged, and the amount of ethylene to be recovered from the pipe increased.

Meanwhile, in Comparative Example 2, the liquid level in the reactor is controlled by discharging the liquid that has risen above the boundary height through the overflow pipe 10 at the boundary between the gas region and the liquid region without separately measuring the liquid level in the reactor 100, and as a result, a large amount of gas stream containing unreacted ethylene was discharged together with the liquid stream through the overflow pipe 10. As a result, it is difficult to predict the content of ethylene discharged through the overflow pipe, and the clogging phenomenon occurred due to the increase in the two-phase of liquid-gas within the pipe.

### [Detailed Description of Main Elements]

- 100:: Reactor
- 200:: Gas-liquid separator
- 10:: Overflow pipe
- 11:: Pressure maintenance pipe
- 20:: Liquid level gauge
- 210:: Liquid discharge pipe

## Claims

1. An apparatus for preparing an oligomer, comprising:
a reactor that includes an upper gas region and a lower liquid region and performs an oligomerization reaction by receiving an ethylene gas and a solvent to the liquid region;
an overflow pipe that is provided on an outer side wall of the reactor and connected to a height corresponding to a boundary between the gas region and the liquid region of the reactor to discharge a liquid stream containing an oligomer product;
a gas-liquid separator that is connected to the overflow pipe and separates gas from the liquid stream;
a liquid level gauge that is provided in the gas-liquid separator; and
a liquid discharge pipe that is connected to a lower portion of the gas-liquid separator and discharges a gas-separated liquid stream.

2. The apparatus of claim 1, wherein the overflow pipe maintains a pressure of the reactor and a pressure of the gas-liquid separator to be the same.

3. The apparatus of claim 1, wherein the liquid stream transferred from the reactor to the gas-liquid separator through the overflow pipe is suppressed accompanying of gas hole up.

4. The apparatus of claim 1, further comprising a pressure maintenance pipe that connects the gas region of the reactor and a gas region of the gas-liquid separator.

5. The apparatus of claim 1, wherein the liquid level gauge measures a liquid level of the gas-separated liquid stream in the gas-liquid separator.

6. The apparatus of claim 5, wherein a measurement value of the liquid level has an error of less than 5% with an actual liquid level in the gas-liquid separator.

7. The apparatus of claim 1, wherein the liquid discharge pipe connected to the lower portion of the gas-liquid separator includes a valve that controls the amount of liquid discharged according to a signal of the liquid level gauge.

8. The apparatus of claim 1, wherein the reactor includes a bubble column reactor.

9. The apparatus of claim 1, wherein the gas region of the reactor includes an upper pipe that discharges a gas stream containing an unreacted ethylene gas.
